# EUROPÄISCHE PATENTSCHRIFT

(11) **EP 3 177 220 B1**
(45) Veröffentlichungstag und Bekanntmachung des Hinweises auf die Patenterteilung: **23.05.2018**
(21) Anmeldenummer: 15741519.1
(22) Anmeldetag: 15.07.2015
(51) Int. Cl.: A61B 17/70

(54) **OSTEOSYNTHESEVORRICHTUNG**
OSTEOSYNTHESIS DEVICE
DISPOSITIF D'OSTÉOSYNTHÈSE

(30) Priorität: 06.08.2014 DE 102014215529
(43) Veröffentlichungstag der Anmeldung: 14.06.2017
(73) Patentinhaber: Silony Medical International AG, 8500 Frauenfeld (CH)
(72) Erfinder: TRAUTWEIN, Frank Thilo, 70794 Filderstadt (DE); HEUER, Frank, 70794 Filderstadt (DE); OHNMACHT, Timo, 78736 Trichtingen (DE)
(74) Vertreter: DREISS Patentanwälte PartG mbB
(86) Internationale Anmeldenummer: PCT/EP2015/066148
(87) Internationale Veröffentlichungsnummer: WO 2016/020158

(56) Entgegenhaltungen:
- US-A1- 2011 160 778
- US-A1- 2011 282 399
- US-A1- 2012 046 699
- US-A1- 2012 310 284

## Beschreibung

Die Erfindung betrifft eine Osteosynthesevorrichtung mit einer einen Gewindeschaft und einen Kugelkopf aufweisenden Knochenschraube und mit einem eine Nut aufweisenden Gabelkopf mit zwei Schenkeln, z.B. für einen in die Nut des Gabelkopfs zwischen die beiden Schenkel einzulegenden Verbindungsstab, wobei der Kugelkopf im Gabelkopf verschwenkbar gelagert ist, und mit einem Druckstück, das auf dem Kugelkopf aufsitzt, wobei sich das Druckstück am Gabelkopf abstützt und zwei seitliche, in Achsrichtung des Gabelkopfes und weg vom Gewindeschaft sich erstreckende Flügel aufweist, wobei das Druckstück unter Vorspannung in Achsrichtung und damit in Richtung auf den Kugelkopf im Gabelkopf gelagert ist, indem wenigstens einer der Flügel quer zur Achsrichtung des Gabelkopfs geschlitzt ist, so dass ein Schlitz eine Federzunge bildet und das Druckstück dadurch federnd ausgebildet ist und sich über die Federzunge am Gabelkopf abstützt. Unter dem Begriff Kugelkopf wird auch ein Schraubenkopf verstanden, der eine nicht exakt kugelförmig ausgebildete Lagerfläche aufweist, solange sie mit dem Gabelkopf nach Art eines Kalottenlagers zusammenwirkt.

Eine gattungsgemäße Osteosynthesevorrichtung ist beispielsweise bekannt aus US 2011/0282399 A1 oder aus US 2012/0046699 A1.

Aus der DE 196 17 362 C2 ist ein Verankerungselement bekannt, das eine Knochenschraube mit einem Schaft und einem Gabelkopf aufweist, wobei in den Gabelkopf ein Verbindungsstab eingelegt und fixiert wird. Außerdem ist ein Einsatzelement vorgesehen, welches ein Auflager für den Verbindungsstab bildet.

Die EP 1 323 391 A2 zeigt eine Polyaxialschraube mit einem Gewindeschaft und einem kugelsegmentförmigen Kopf, der in einem Gabelkopf gelagert ist. Auf diesem Kugelkopf sitzt ein Druckstück auf, welches auch ein Auflager für den Verbindungsstab bildet. Das Druckstück wird mit einer in den Gabelkopf eingeschraubten Schraubhülse gegen den Kugelkopf gedrückt.

Aus der WO 2004/098423 A1 ist eine Polyaxialschraube bekannt, bei welcher der im Gabelkopf liegende Kugelkopf der Knochenschraube vom Verbindungsstab elastisch in die ihn aufnehmende Kalotte gedrückt wird. Ohne Verbindungsstab übt das Verbindungselement keine Kräfte auf den Kugelkopf aus.

Aus US 2006/0241599 A1 ist eine Polyaxialschraube bekannt, bei welcher ein Druckstück in der Längsachsrichtung erstreckte Schlitze aufweist, so dass ein dazwischenliegender Wandbereich des Druckstücks durch eine nach radial innen gerichtete Deformation des Gabelkopfs ebenfalls nach radial innen auslenkbar ist und dabei eine Klemmkraft auf den Kugelkopf der Knochenschraube ausübt.

Aus US 2010/0137920 A1 ist eine Polyaxialschraube bekannt, bei welcher ein Druckstück in der Längsachsrichtung über Schraubenfedern gegen den Kugelkopf der Knochenschraube kraftbeaufschlagt ist.

Weitere Osteosynthesevorrichtungen sind bekannt aus DE 60 2005 002 477 T2 und US 2011/0077694 A1.

Der Erfindung liegt die Aufgabe zugrunde, eine Osteosynthesevorrichtung der eingangs genannten Art derart weiterzubilden, dass sie bei der chirurgischen Implantation komfortabler handhabbar ist.

Diese Aufgabe wird bei einer Osteosynthesevorrichtung der eingangs genannten Art nach einer ersten Variante der Erfindung (Anspruch 1) dadurch gelöst, dass ein Vorsprung in der Achsrichtung in den Schlitz einragt und dass der Vorsprung zur Begrenzung einer Verformung oder Auslenkung der Federzunge einen in der Achsrichtung wirkenden Anschlag für die Federzunge bildet.

Das Druckstück ist also einerseits gegen den Gabelkopf in der Achsrichtung abgestützt und drückt andererseits in Folge seiner federnden Ausbildung auf den Kugelkopf der Knochenschraube. Da sich das Druckstück unter axialer Vorspannung einerseits am Gabelkopf und andererseits am Kugelkopf der Knochenschraube abstützt, bedarf es keines Verbindungsstabs oder eines anderen Mittels, um den Gabelkopf relativ zu dem Kugelkopf und Gewindeschaft so vorläufig zu fixieren, dass eine unerwünschte Verschwenkung des Gabelkopfs gegenüber dem Kugelkopf verhindert oder zumindest erschwert wird. Durch die axiale Vorspannung des Druckstücks wird also der Kugelkopf der Knochenschraube gegenüber einer kalottenartigen Lagerfläche am Gabelkopf weitestgehend unverstellbar gestellt. Dies kann schon hilfreich sein, wenn die Knochenschraube eingeschraubt wird.

Allein der korrekte Sitz des Druckstücks am Gabelkopf gewährleistet den Kraftschluss zwischen Knochenschraube und Druckstück und damit eine Vorfixierung zwischen Knochenschraube und Gabelkopf vermittels des Druckstücks. Der Gabelkopf und die Knochenschraube können also in einer gewünschten Weise oder Orientierung zueinander eingestellt werden, und behalten diese Orientierung bei, was eine wesentliche Erleichterung für den Chirurgen beim Implantieren und Komplettieren der gesamten Osteosynthesevorrichtung bedeutet.

Das Druckstück weist zwei seitliche, in Achsrichtung des Gabelkopfs und weg vom Gewindeschaft sich erstreckende Flügel auf. Diese Flügel erstrecken sich bei in den Gabelkopf eingesetztem Druckstück insbesondere parallel zu den Schenkeln des Gabelkopfs. Eine der Aufgaben der Flügel besteht darin, das Druckstück am Gabelkopf abzustützen, so dass Kräfte zwischen dem Druckstück und dem Gabelkopf übertragen werden können. Dabei sollen Kräfte in der Achsrichtung des Gabelkopfs auf den Kugelkopf der Knochenschraube übertragen werden, der in der kalottenartigen Lagerfläche des Gabelkopfs gelagert ist.

Die erfindungsgemäße Osteosynthesevorrichtung besitzt zum einen den wesentlichen Vorteil, dass die Knochenschraube mit deren Kugelkopf kraftschlüssig im Gabelkopf gelagert ist, so dass der Gabelkopf durch den Chirurgen vor dem Einschrauben oder erst nach dem Einschrauben in den Knochen gegenüber dem Kugelkopf ausgerichtet werden kann. Er kann dann nicht seitlich wegkippen, so dass alle Gabelköpfe ausgerichtet werden können und ausgerichtet bleiben, damit ein Verbindungs- oder Korrekturstab gewissermaßen gleichzeitig in die Gabelköpfe eingelegt werden kann. Die erfindungsgemäße Osteosynthesevorrichtung erleichtert also die Implantation der Osteosythesevorrichtung, die zudem schneller durchgeführt werden kann.

Erfindungsgemäß wird durch die geschlitzte Ausbildung wenigstens eines Flügels des Druckstücks und durch die hiermit einhergehende Ausbildung einer Federzunge erreicht, dass die vorstehend erwähnte in axialer Richtung wirkende Vorspannung durch geringfügiges axiales Zusammendrücken des Druckstücks unmittelbar realisiert werden kann. Es wird also erfindungsgemäß innerhalb des Druckstücks, d.h. innerhalb wenigstens eines Flügels des Druckstücks, eine axiale, d.h. in Achsrichtung des Gabelkopfs wirkende elastische Nachgiebigkeit erreicht, so dass beim Einklipsen oder Einrasten oder beim Verdrehen des Druckstücks in eine Montageposition am Gabelkopf eine axiale Vorspannung in Achsrichtung des Gabelkopfs und damit in Richtung auf den Kugelkopf erzielt.

Des Weiteren wird erfindungsgemäß vorgeschlagen, dass die Verformung oder Auslenkung der Federzunge beim Einklipsen oder Einrasten oder Eindrehen des Druckstücks in seine Montageposition begrenzt wird, und zwar durch einen in der Achsrichtung in den Schlitz einragenden Vorsprung, der hierbei einen Anschlag für die Federzunge bildet. Auf diese Weise kann erfindungsgemäß verhindert werden, dass sich in der Federzunge, insbesondere im Bereich der Anlenkung der Federzunge an den feststehenden Teil des Druckstücks zu starke Kräfte im Zuge der Auslenkung der Federzunge einstellen, die eine plastische Verformung des Druckstücks hervorrufen könnten, was sich als nachteilig erweisen könnte.

Der genannte in den Schlitz einragende Vorsprung kann nach einer Ausführungsform der Erfindung in der Achsrichtung gegenüberliegend der Federzunge am Druckstück ausgebildet sein und dort insbesondere einstückig mit dem Druckstück ausgebildet sein. Er kann nach einer weiteren Ausführungsform der Erfindung auch an der Federzunge ausgebildet sein, insbesondere einstückig mit der Federzunge ausgebildet sein.

Es erweist sich weiter als vorteilhaft, wenn der Vorsprung an einem freien Ende der Federzunge oder an einem dem freien Ende der Federzunge in Achsrichtung gegenüberliegenden Bereich am Druckstück ausgebildet ist. Solchenfalls wird beim Anliegen des Vorsprungs eine langlochartige Öffnung zwischen der Federzunge und dem übrigen Teil des Druckstücks gebildet.

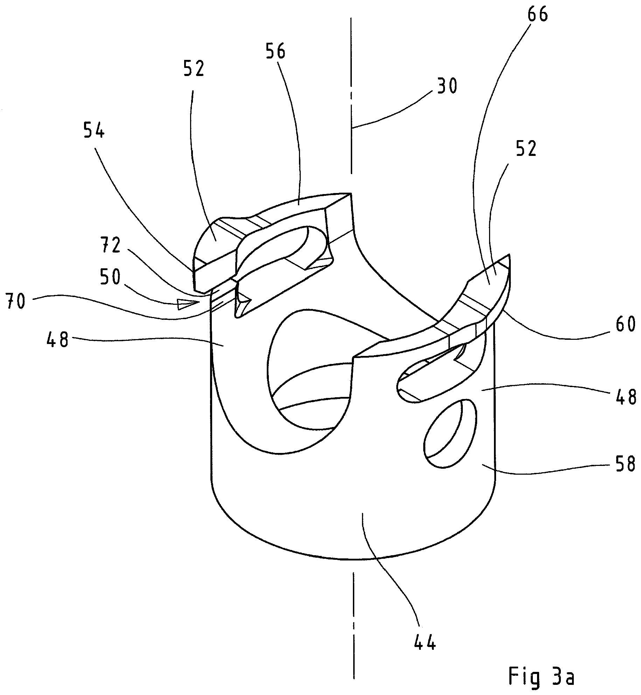

Wie erwähnt, kann das Druckstück an einem oder an beiden Flügeln geschlitzt ausgebildet sein. Im letztgenannten Fall, der als bevorzugt angesehen wird, kann es sich als vorteilhaft erweisen, wenn das Druckstück und dessen Flügel zur Achsrichtung des Gabelkopfs achssymmetrisch oder in anderen Worten rotationssymmetrisch ausgebildet sind. Eine solche Ausbildung zeigt Figur 3a.

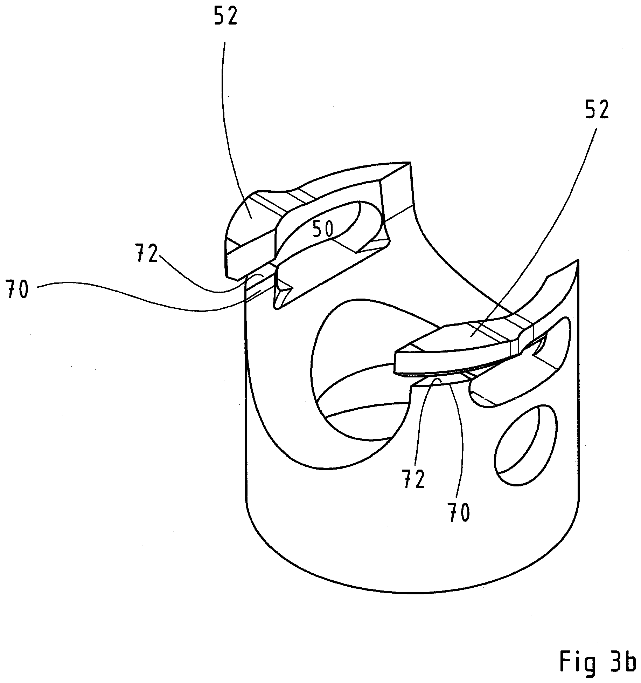

Ebenso kann es sich als vorteilhaft erweisen, wenn das Druckstück und dessen Flügel zu einer die Achsrichtung des Gabelkopfs einschließenden Ebene spiegelsymmetrisch ausgebildet sind. Eine solche Ausführungsform zeigt Figur 3b.

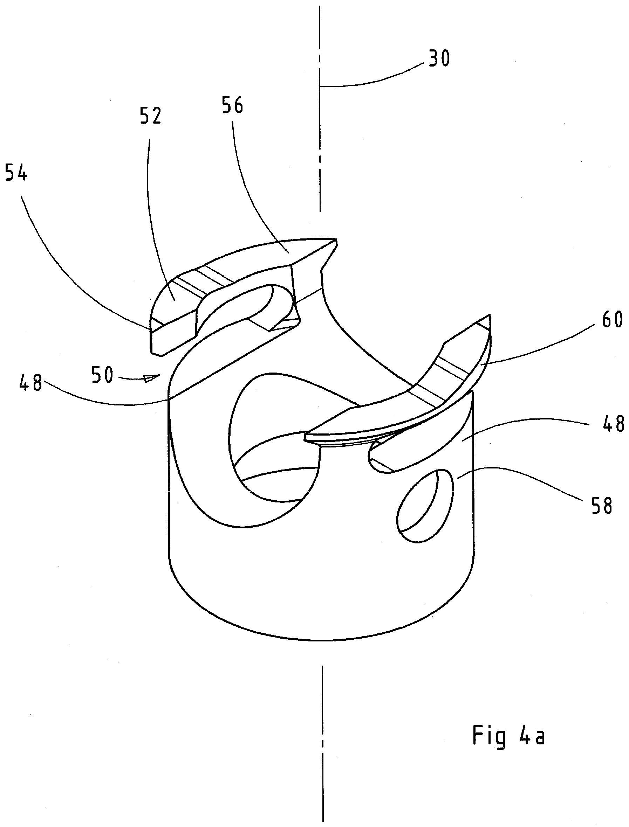

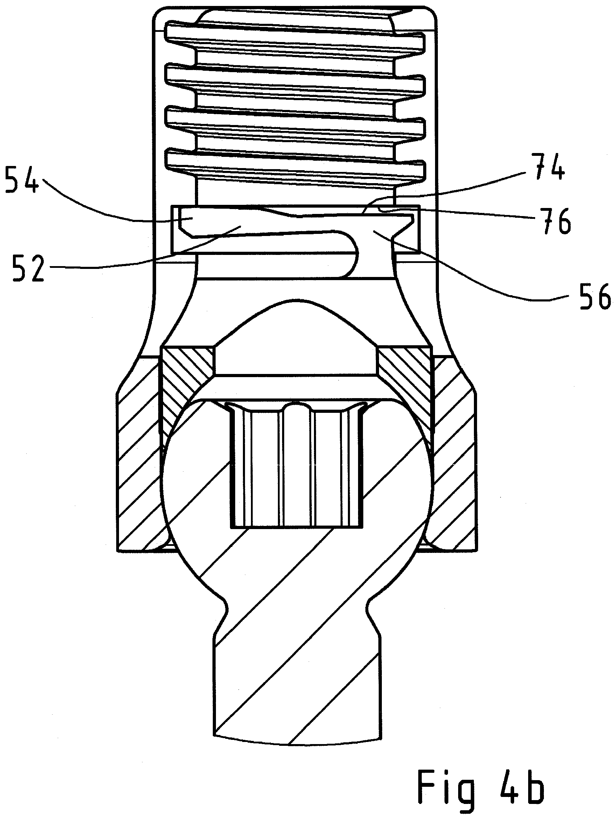

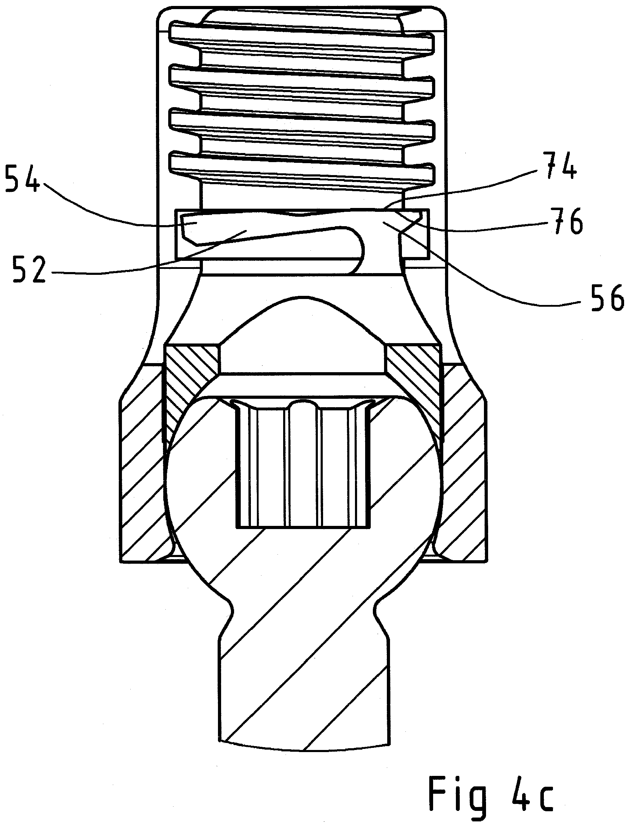

Die obige Aufgabe wird nach einer zweiten Variante der Erfindung (Anspruch 5) dadurch gelöst, dass die Federzunge im an den Gabelkopf montierten jedoch nicht zusätzlich axial belasteten Zustand im Bereich ihres an den Flügel angebundenen Endes in Achsrichtung vom Gabelkopf beabstandet ist und dass die Federzunge im zusätzlich axial belasteten Zustand im Bereich ihres an den Flügel angebundenen Endes einen Anschlag bildet und mit diesem Anschlag zur Begrenzung der weitergehenden Verformung oder Auslenkung der Federzunge gegen den Gabelkopf abstützbar ist. Diese Variante der Erfindung ist in Figuren 4 a-c dargestellt. Bei beiden Varianten wird eine zu weit gehende Verformung der Federzunge durch Ausbildung eines die Auslenkung der Federzunge begrenzenden Anschlags verhindert.

Im an den Gabelkopf montierten jedoch nicht zusätzlich axial belasteten Zustand stützt sich die Federzunge vorzugsweise im Bereich ihres freien Endes gegen den Gabelkopf ab.

Weiter erweist es sich bei beiden Varianten der Erfindung als vorteilhaft, wenn der Schlitz an seinem geschlossenen Ende, also dort, wo die Federzunge in den übrigen Teil des Druckstücks übergeht, verrundet ausgebildet ist. Auch am anderen offenen Ende kann es sich als vorteilhaft erweisen, wenn der dort vorgesehene Vorsprung verrundet in die Federzunge oder in den feststehenden Teil des Druckstücks übergeht.

Es hat sich weiter als vorteilhaft erwiesen, wenn die Auslenkung der Federzunge beim Einklipsen oder Einrasten oder Eindrehen des Druckstücks in seine Montageposition im Gabelkopf höchstens 1,0 mm, insbesondere höchstens 0,5 mm und weiter insbesondere wenigstens 0,1 mm beträgt. Auf diese Weise wird sicher verhindert, dass zu starke Spannungen innerhalb des Druckstücks entstehen, die zu plastischen Deformationen des Druckstücks führen könnten.

Im Hinblick auf eine einfache, betriebssichere und für den Chirurgen zweckmäßig ausführbare Montage des Druckstücks am Gabelkopf erweist es sich als vorteilhaft, wenn an Außenseiten der Flügel des Druckstücks Vorsprünge oder Ausnehmungen vorhanden sind, die mit Ausnehmungen oder Vorsprüngen an Innenflächen der Schenkel des Gabelkopfs ganz oder teilweise ineinandergreifend zusammenwirken, also das Druckstück unter axialer Vorspannung am Gabelkopf halten, so dass es auf den Kugelkopf drückt und so den Gabelkopf gegenüber dem Kugelkopf und gegenüber dem Gewindeschaft kraftschlüssig vorfixiert.

Hierfür wird nach einer Ausführungsform der Erfindung vorgeschlagen, dass das Druckstück eine an einer Innenseite eines Schenkels des Gabelkopfs vorhandenen Abstufung untergreift oder in eine dort ausgebildete Ausnehmung eingreift. Hierfür kann das Druckstück insbesondere an seinem vom Kugelkopf abgewandten Ende einen in radialer Richtung vorstehenden Rand oder auch nur einen oder mehrere radial vorstehende Ansätze aufweisen, die dann mit einer solchen Abstufung oder Ausnehmung am Gabelkopf verklipsbar oder verrastbar oder in diese eindrehbar sind, wenn das Druckstück in seine Montageposition am Gabelkopf gebracht wird, wobei dann die durch den Schlitz gebildete Federzunge geringfügig ausgelenkt wird. Vorzugsweise wird das Druckstück in Achsrichtung des Gabelkopfs in den Gabelkopf eingeführt und dann um die Achsrichtung in seine Montageposition gedreht.

Des Weiteren kann es sich als vorteilhaft erweisen, dass ein Flügel oder beide Flügel des Druckstücks mehrfach geschlitzt ausgebildet ist/sind, also mehrere Schlitze aufweist/en, die abwechselnd von einer Seite und von einer dieser gegenüberliegenden Seite quer zur Achsrichtung des Gabelkopfs in den Flügel einschneiden. Es werden so entsprechend mehrere Federzungen gebildet.

Das Druckstück der erfindungsgemäßen Osteosynthesevorrichtung kann unmittelbar oder mittelbar ein Auflager für einen Verbindungsstab bilden. Nach dem Fixieren des Verbindungsstabs durch Festziehen eines zusätzlichen Fixiermittels, insbesondere einer Madenschraube, in ein Gewinde am freien Ende des Gabelkopfs drückt der Verbindungsstab über das Druckstück auf den Kugelkopf und die Lagerfläche des Gabelkopfs, so dass alle Komponenten am Ende der Operation miteinander dauerhaft verspannt und fixiert werden. Die im Druckstück zuvor ausgebildete axiale Vorspannung hat dann praktisch keine Funktion mehr.

Nach einem weiteren Erfindungsgedanken erweist es sich als vorteilhaft, wenn das Druckstück gegenüber dem Gabelkopf verdrehgesichert ist, also um die Achsrichtung des Gabelkopfs nicht rotierbar ist. Dies kann beispielsweise durch eine parallel zur Achsrichtung verlaufende Längs- oder Schiebesitzführung oder in anderer Weise realisiert werden.

Weiter erweist sich eine Ausführungsform als vorteilhaft, bei der die Federzunge am freien Ende in radialer und/oder in axialer Richtung dicker ausgebildet ist als am geschlossenen Ende.

Losgelöst von der vorausgehend beschriebenen erfindungsgemäßen Begrenzung der Auslenkung der Federzunge durch einen in den Schlitz ragenden Vorsprung wird selbständiger Schutz in Anspruch genommen für eine Osteosynthesevorrichtung mit einer einen Gewindeschaft und einen Kugelkopf aufweisenden Knochenschraube und mit einem eine Nut aufweisenden Gabelkopf mit zwei Schenkeln, wobei der Kugelkopf im Gabelkopf verschwenkbar gelagert ist, und mit einem Druckstück, das auf dem Kugelkopf aufsitzt, wobei sich das Druckstück am Gabelkopf abstützt und zwei seitliche, in Achsrichtung des Gabelkopfes und weg vom Gewindeschaft sich erstreckende Flügel aufweist, wobei die Osteosynthesevorrichtung dadurch gekennzeichnet ist, dass das Druckstück unter Vorspannung in Achsrichtung und damit in Richtung auf den Kugelkopf im Gabelkopf gelagert ist, indem wenigstens einer der Flügel quer zur Achsrichtung des Gabelkopfs geschlitzt ist, so dass ein Schlitz eine Federzunge bildet und das Druckstück dadurch federnd ausgebildet ist. Das Druckstück ist also einerseits gegen den Gabelkopf in der Achsrichtung abgestützt und drückt andererseits in Folge seiner federnden Ausbildung auf den Kugelkopf der Knochenschraube.

Weitere Merkmale, Einzelheiten und Vorteile der Erfindung ergeben sich aus den beigefügten Patentansprüchen und der zeichnerischen Darstellung und nachfolgenden Beschreibung bevorzugter Ausführungsformen der.

In der Zeichnung zeigt:
Figuren 1a, b eine insgesamt mit dem Bezugszeichen 10 bezeichnete erfindungsgemäße Osteosynthesevorrichtung und Detailansicht;
Figuren 2a, b perspektivische Teilansichten eines Gabelkopfs mit Knochenschraube der erfindungsgemäßen Osteosynthesevorrichtung mit Schnittansicht;
Figuren 2c, d perspektivische Teilansichten des Gabelkopfs mit Knochenschraube gemäß Figur 2a und mit eingesetztem Druckstück;
Figuren 3a-c perspektivische Ansichten dreier Ausführungsformen eines Druckstücks der erfindungsgemäßen Osteosynthesevorrichtung gemäß einer ersten Variante der Erfindung;
Figuren 4a-c perspektivische Ansichten und teilweise Schnittansichten einer weiteren Ausführungsformen der erfindungsgemäßen Osteosynthesevorrichtung gemäß einer zweiten Variante der Erfindung.

Die Osteosynthesevorrichtung umfasst eine Knochenschaube 12, einen Gabelkopf 14, ein Druckstück 16, ein lediglich andeutungsweise dargestelltes Korrektur-, Stütz- oder Verbindungsmittel oder -Stab 18 und ein Klemmmittel 20, das beispielhaft in Form einer Madenschraube 22 ausgebildet ist. Bei der hier in Rede stehenden Erfindung geht es um das Zusammenwirken des Druckstücks 16 mit dem Gabelkopf 14 und der Knochenschraube 12. Die Knochenschraube 12 besitzt einen Gewindeschaft 24 sowie einen Kugelkopf 26, die im nur beispielhaft dargestellten Fall einstückig ausgebildet sind, jedoch auch von zwei miteinander gefügten Teilen gebildet werden können. Der Kugelkopf 26 ist in einer Kalotte 28 des Gabelkopfs 14 gelenkig oder verschwenkbar nach Art eines Kalottenlagers aufgenommen und kann diesbezüglich in noch zu beschreibender Weise kraftschlüssig unverschwenkbar gestellt werden.

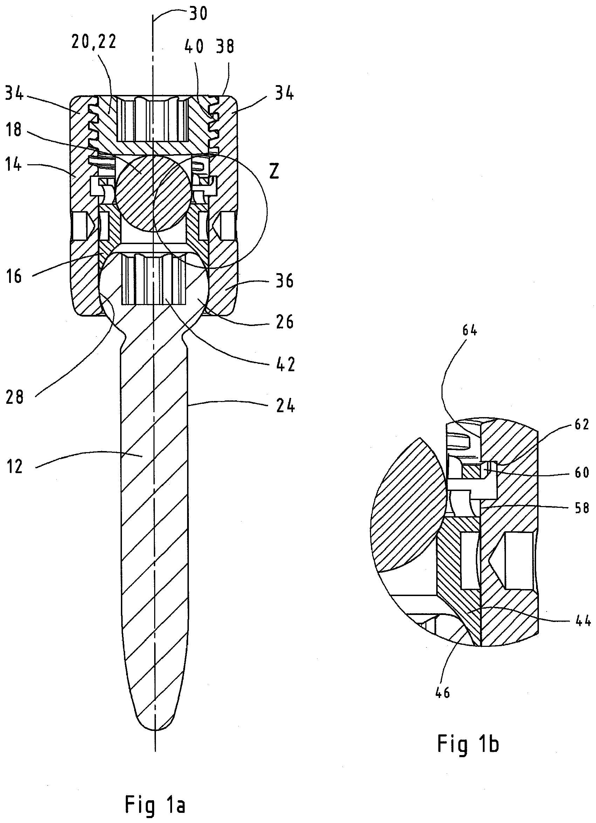

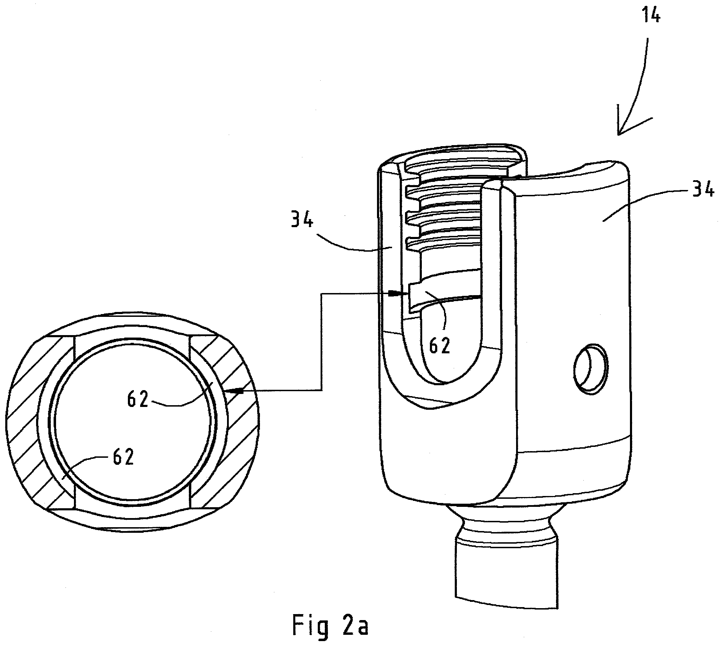

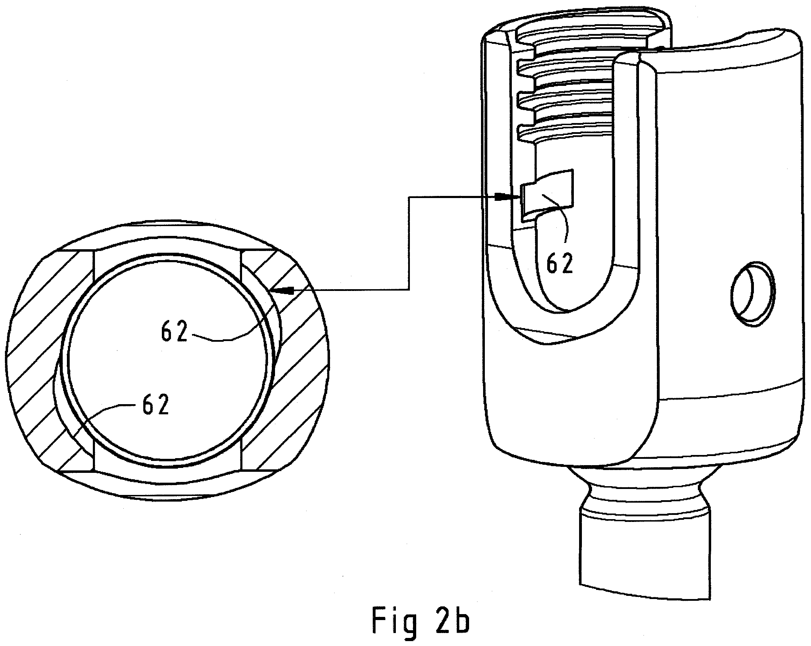

Der Gabelkopf 14, der auch perspektivisch in Figuren 2a und 2b dargestellt ist, ist ungefähr zylindrisch oder topfförmig ausgebildet, wobei die Zylinder- oder Topfform derart unterbrochen ist, das eine quer zur Achsrichtung 30 verlaufende Nut 32 zum Einlegen des Korrektur-, Stütz- oder Verbindungsmittels 18 gebildet wird. Diese Nut 32 ist in Umfangsrichtung begrenzt durch zwei in Achsrichtung 30 erstreckte und konzentrisch ausgebildete Schenkel 34, die einander diametral zur Achsrichtung 30 gegenüberliegen. Die Schenkel 34 sind nach unten hin, also in Richtung auf den Kugelkopf 26, durch einen in Umfangsrichtung geschlossenen, insbesondere ring- oder zylinderförmigen Basisbereich 36 verbunden und vorzugsweise einstückig mit diesem ausgebildet. Die Schenkel 34 tragen ausgehend von ihrem freien Ende 38 ein Innengewinde 40, in welches die Madenschraube 22 einschraubbar ist. Bevor die Madenschraube 22 zur Klemmung des Korrektur-, Stütz- oder Verbindungsmittels 18 in den Gabelkopf 14 eingeschraubt wird, muss jedoch zunächst die Knochenschraube 12 in den Knochen eingeschraubt werden. Hierfür greift der Chirurg von oben mit einem geeigneten Werkzeug zwischen den Schenkeln 34 des Gabelkopfs 14 hindurch und setzt das nicht dargestellte Werkzeug in oder an eine am Kugelkopf 26 ausgebildete Werkzeugansetzstelle 42 ein oder an. Um währenddessen den Gabelkopf 14 in einer geeigneten Position und im Wesentlichen starr am Kugelkopf 26 zu halten, wird das Druckstück 16 verwendet. Das in Figur 1a verbaut dargestellte Druckstück 16 ist in Figur 3a perspektivisch dargestellt. Das Druckstück 16 umfasst zum Kugelkopf 26 hin einen Druckring 44 mit einer dem Kugelkopf 26 zugewandten und gegen diesen nach Art eines Kalottenlagers anlegbaren Gleitfläche 46 (s. Figur 1b). Ausgehend von dem Druckring 44 erstrecken sich auf diametral gegenüberliegenden Seiten bezüglich der Achsrichtung 30 zwei Flügel 48. Wenigstens einer der Flügel 48 ist quer zur Achsrichtung 30 geschlitzt ausgebildet. Auf diese Weise bildet ein hierdurch gebildeter Schlitz 50 eine Federzunge 52. Die Federzunge 52 ist also ein Teil des betreffenden Flügels 48 und wird durch die geschlitzte Ausbildung dieses Flügels 48 quer zur Achsrichtung 30 begrenzt und dadurch gebildet. Die Federzunge 32 umfasst ein freies Ende 54 und ein Ende 56, mit dem sie an den Rest des jeweiligen Flügels 48 angebunden bleibt. Die Federzunge 52 kann axial, also in Achsrichtung 30, ausgelenkt werden, so dass der jeweilige Flügel 48 und dadurch das Druckstück 16 insgesamt unter Vorspannung in Achsrichtung 30 bringbar ist. Mittels dieser Vorspannung kann sich das Druckstück 16 einerseits gegen den Gabelkopf 14 auf noch näher zu beschreibende Weise und andererseits gegen den Kugelkopf 26 abstützen. Auf diese Weise kann der Kugelkopf und damit der Gewindeschaft 24 gegeneinander kraftschlüssig unverschwenkbar gestellt werden.

Dies geschieht nach einer Ausführungsform der Erfindung dadurch, dass an einer Außenseite 58 eines Flügels 48 Vorsprünge 60 oder Ausnehmungen vorgesehen sind, die mit Ausnehmungen 62 oder Vorsprüngen an einer Innenfläche 64 der Schenkel 34 des Gabelkopfs dahingehend komplementär zusammenwirken, dass eine Auslenkung der Federzunge 52 in axialer Richtung und damit eine Vorspannung in der Achsrichtung 30 innerhalb des Druckstücks 16 erzeugt wird. Mittels dieser axialen Vorspannung drückt dann das Druckstück 16 mit seiner Gleitfläche 46 gegen den Kugelkopf 26 und drückt diesen wiederum gegen die Kalotte 28 des Gabelkopfs 14, so dass diese Komponenten gegeneinander unverschwenkbar gestellt sind.

Ein solcher Vorsprung in radialer Richtung über die Außenseite 58 des Flügels 48 des Druckstücks ist indessen nicht zwingend erforderlich; es wäre insbesondere denkbar, dass das Druckstück mit seiner axialen Stirnseite 66 eine Abstufung an der Innenseite oder Innenfläche 64 des Schenkels 34 des Gabelkopfs 14 untergreift, wobei diese Abstufung dann nach radial innen vorsteht. Beispielsweise könnte das Druckstück dann durch Drehen um die Achsrichtung 30 in diese Anlageposition verdreht werden. D. h. das Druckstück 16 wird in einer ersten Drehposition in den Gabelkopf 14 axial eingesetzt und dann um die Achsrichtung 30 verdreht, so dass die Federzunge 52 axial mit einem Vorsprung oder einer Abstufung in den betreffenden Schenkel 34 des Gabelkopfs in Anlage kommt und hierdurch axial ausgelenkt wird, so dass die axiale Vorspannung in dem Druckstück 16 aufgebaut wird.

Bei dem in Figuren 1 bis 3 dargestellten Ausführungsbeispiel umfassen beispielhaft beide Schenkel 34 des Gabelkopfs 14 eine beispielhaft horizontal verlaufende nutförmige Ausnehmung 62, in welche die jeweilige Federzunge 52 eingreift, wobei die Federzunge 52 über die Außenseite 58 des jeweiligen Flügels 48 radial vorsteht. Dieser komplementäre Eingriff, mittels dessen auch die axiale Auslenkung der jeweiligen Federzunge 52 bewirkt wird, könnte dadurch hergestellt werden, dass das Druckstück 16 in Achsrichtung 30 zwischen die Schenkel 34 des Gabelkopfs 14 eingeführt wird, wobei die Federzungen 52 in die in Figur 1b dargestellte Montageposition einrasten oder eingeklipst werden. Es ist jedoch auch denkbar und vorteilhaft, dass das Druckstück 16 zunächst in einer ersten Drehstellung, in der die Flügel im Bereich der Nut 32 zwischen den Schenkeln 34 des Gabelkopfs 14 orientiert sind, in Achsrichtung 30 in den Gabelkopf 14 eingeführt wird und dann in die in den Figuren dargestellte bestimmungsgemäße Montageposition um die Achsrichtung 30 verdreht wird. Es besteht dann nicht die Gefahr, dass die Federzungen vor dem Einrasten oder Einklipsen übermäßig in die andere Richtung ausgelenkt werden.

Die Figuren 2a und 2b zeigen unterschiedliche Ausbildungen der nutförmigen Ausnehmung 62, die beide für das in Figur 3a dargestellte Druckstück 16 geeignet sind. Bei Figur 2a ist die nutförmige Ausnehmung 62 über die gesamte Umfangserstreckung des jeweiligen Schenkels 34 erstreckt, und in Figur 2b ist sie nur bereichsweise erstreckt.

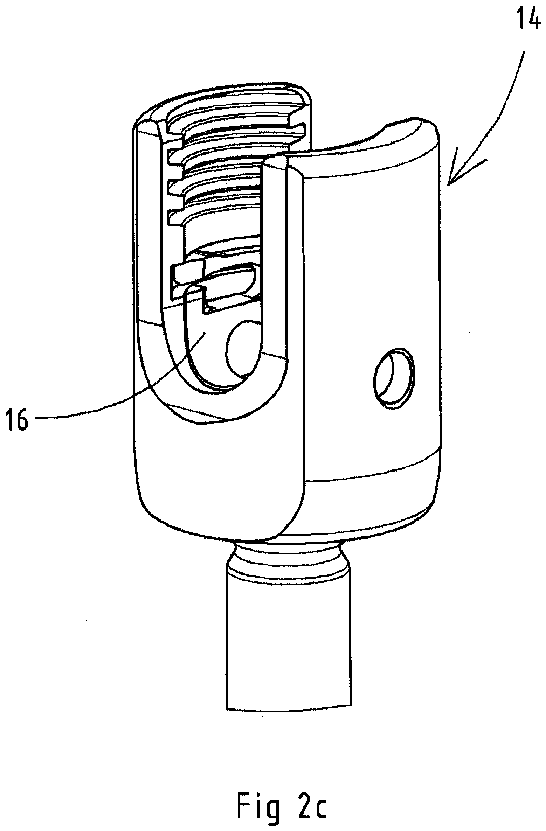

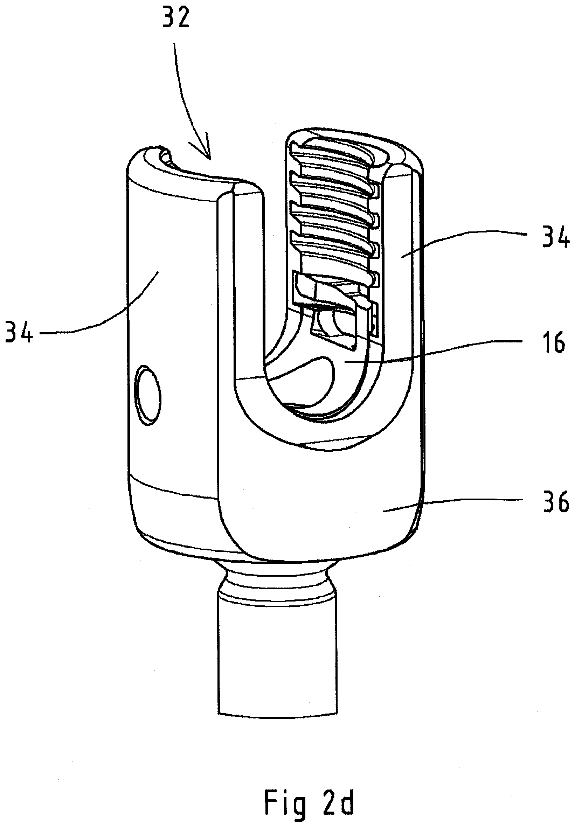

Die Figuren 2c und 2d zeigen die erfindungsgemäße Osteosynthesevorrichtung mit in den Gabelkopf eingesetztem Druckstück 16 gemäß Figur 3a.

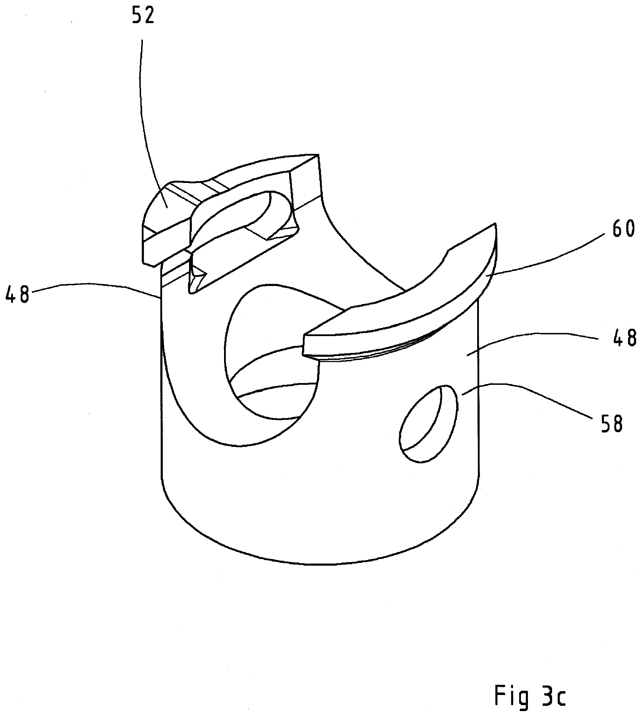

Schließlich zeigen die Figuren 3b und 3c weitere Ausführungsformen des Druckstücks 16, die sich von der in Figur 3a dargestellten rotationssymmetrischen Ausbildung des Druckstücks unterscheiden. Bei Figur 3b sind die Federzungen 52 bezüglich einer die Achsrichtung 30 einschließenden Ebene spiegelsymmetrisch ausgebildet und am Druckstück 16 angeordnet. Bei der Ausführungsform gemäß Figur 3c ist nur an einem Flügel 48 eine Federzunge 52 ausgebildet. Bei dem gegenüberliegenden Flügel 48 ist an dessen freiem Ende jedoch auch ein radialer Überstand über die Außenseite 58 dieses Flügels 48 ausgebildet, der einen Vorsprung 60 bildet und in eine nutförmige Ausnehmung 62 im zugeordneten Schenkel 34 des Gabelkopfs eingreifen kann. Es hat sich gezeigt, dass auch bei einseitiger Ausbildung einer Federzunge 52 eine hinreichende axiale Vorspannung in dem Druckstück 16 erreicht werden kann, um den Gabelkopf 14 gegenüber dem Kugelkopf 26 der Knochenschraube 12 unverschwenkbar zu stellen, so dass beim Einschrauben der Knochenschraube 12 in den Knochen der Gabelkopf 14 in einer geeigneten Position gehalten werden kann.

Weiter ist nach einer ersten Variante der Erfindung vorgesehen, dass bei allen Druckstücken 16 gemäß Figuren 3a-c ein Vorsprung 70 vorgesehen ist, der in Achsrichtung 30 in den Schlitz 50 einragt und so als Anschlag 72 zur Begrenzung einer Auslenkung der Federzunge 52 dient. Die jeweilige Federzunge 52 kann also axial nur so weit ausgelenkt werden bis sie axial gegen diesen Vorsprung 70 oder Anschlag 72 anliegt. Bei den beispielhaft dargestellten Ausführungsformen ist dieser Vorsprung 70 an den feststehenden Teil des jeweiligen Flügels 48 des Druckstücks 16 angeformt. Es wäre indessen auch möglich, dass der Vorsprung an die bewegliche Federzunge 52 angeformt ist. Dieser Vorsprung 70 und die hierdurch erfindungsgemäß herbeigeführte Begrenzung der Verformung oder Auslenkung der jeweiligen Federzunge 52 erweist sich als besonders vorteilhaft. Wenn nämlich im Zuge der Handhabung beim Implantieren der Osteosynthesevorrichtung, insbesondere beim Einschrauben der Knochenschraube 12, von oben Druck auf den Gabelkopf 14 ausgeübt wird, so drückt der Kugelkopf 26 von unten gegen das Druckstück 16 und würde ohne Begrenzung der Auslenkung der Federzungen 52 das Druckstück 16 axial nach oben drücken und so die Gefahr einer plastischen Deformation der Federzungen 52 hervorrufen. Durch den erfindungsgemäß vorgesehenen Vorsprung 70, der einen Anschlag 72 für die Auslenkung der Federzunge 52 bildet, kann dies aber nicht oder in geringerem Maße geschehen.

Die Figuren 4a-c zeigen eine Ausführungsform der Osteosynthesevorrichtung nach einer zweiten Variante der Erfindung. Wie bei den Druckstücken gemäß Figuren 3a-c ist eine Federzunge 52 gebildet, indem in dem betreffenden Flügel 48 ein Schlitz 50 ausgebildet ist. Allerdings wird bei diesen Ausführungsformen gemäß Figuren 4a-c eine Begrenzung der Auslenkung der Federzungen 52 nicht durch einen in den Schlitz 50 einragenden Vorsprung gebildet, sondern wie aus Figuren 4b und c ersichtlich ist bildet die Federzunge 52 selbst im Bereich ihres an den Flügel 48 angebundenen Endes 56 einen Anschlag 74 zur Begrenzung einer weiteren Auslenkung der Federzunge 52. Dies wird erfindungsgemäß dadurch realisiert, dass die Federzunge 52 im an den Gabelkopf 14 montierten und auf dem Kugelkopf aufsitzenden jedoch nicht zusätzlich axial belasteten Zustand im Bereich ihres an den Flügel 48 angebundenen Endes 56 in Achsrichtung 30 vom Gabelkopf 14 beabstandet ist, also nicht gegen eine axiale Stützfläche 76, einen Vorsprung, eine Ausnehmung oder eine sonstige beliebige axiale Anlagefläche am Gabelkopf 14 anliegt. Dennoch ist das Druckstück 16 in diesem Zustand unter Vorspannung am Gabelkopf 14 gehalten, indem sich die Federzunge 52 hier beispielhaft im Bereich ihres freien Endes 54 gegen den Gabelkopf 14, und zwar gegen die axiale Stützfläche 76 des Gabelkopfs abstützt. Wenn nun - wie in Figur 4c angedeutet - eine zusätzliche axial wirkende Kraft zwischen Kugelkopf und Gabelkopf wirkt, so wird die Federzunge 52 weiter ausgelenkt bis der Anschlag 74 im Bereich des an Flügel 48 angebundenen Endes 56 der Federzunge 52 gegen die axiale Stützfläche 76 des Gabelkopfs anschlägt und eine weitergehende Verformung begrenzt. Bei dieser Ausführungsform gemäß der zweiten Variante der Erfindung ist ein in den Schlitz 50 einragender Vorsprung nicht erforderlich; er könnte aber zusätzlich vorgesehen sein. In entsprechender Weise könnte bei einem Druckstück gemäß der ersten Variante der Erfindung zusätzlich das an den Flügel angebundene Ende der Federzunge als Anschlag gegenüber dem Gabelkopf fungieren.

## Patentansprüche

1. Osteosynthesevorrichtung mit einer einen Gewindeschaft (24) und einen Kugelkopf (26) aufweisenden Knochenschraube (12) und mit einem eine Nut (32) aufweisenden Gabelkopf (14) mit zwei Schenkeln (34), wobei der Kugelkopf (26) im Gabelkopf (14) verschwenkbar gelagert ist, und mit einem Druckstück (16), das auf dem Kugelkopf (26) aufsitzt, wobei sich das Druckstück (16) am Gabelkopf (14) abstützt und zwei seitliche, in Achsrichtung (30) des Gabelkopfs (14) und weg vom Gewindeschaft (24) sich erstreckende Flügel (48) aufweist, wobei das Druckstück (16) unter Vorspannung in Achsrichtung (30) und damit in Richtung auf den Kugelkopf (26) im Gabelkopf (14) gelagert ist, indem wenigstens einer der Flügel (48) quer zur Achsrichtung (30) des Gabelkopfs (14) geschlitzt ist, so dass ein Schlitz (50) eine Federzunge (52) bildet und das Druckstück (16) dadurch federnd ausgebildet ist und sich über die Federzunge (52) am Gabelkopf (14) abstützt, **dadurch gekennzeichnet, dass** ein Vorsprung (70) in der Achsrichtung (30) in den Schlitz (50) einragt und dass der Vorsprung (70) zur Begrenzung einer Verformung oder Auslenkung der Federzunge (52) einen in der Achsrichtung (30) wirkenden Anschlag (72) für die Federzunge (52) bildet.

2. Osteosynthesevorrichtung nach Anspruch 1, **dadurch gekennzeichnet, dass** der Vorsprung (70) in der Achsrichtung (30) gegenüberliegend der Federzunge (52) am Druckstück (16) ausgebildet ist.

3. Osteosynthesevorrichtung nach Anspruch 1, **dadurch gekennzeichnet, dass** der Vorsprung (70) an der Federzunge (52) ausgebildet ist.

4. Osteosynthesevorrichtung nach Anspruch 1, 2 oder 3, **dadurch gekennzeichnet, dass** der Vorsprung (70) an einem freien Ende (54) der Federzunge (52) oder an einem dem freien Ende der Federzunge in Achsrichtung gegenüberliegenden Bereich am Druckstück ausgebildet ist.

5. Osteosynthesevorrichtung mit einer einen Gewindeschaft (24) und einen Kugelkopf (26) aufweisenden Knochenschraube (12) und mit einem eine Nut (32) aufweisenden Gabelkopf (14) mit zwei Schenkeln (34), wobei der Kugelkopf (26) im Gabelkopf (14) verschwenkbar gelagert ist, und mit einem Druckstück (16), das auf dem Kugelkopf (26) aufsitzt, wobei sich das Druckstück (16) am Gabelkopf (14) abstützt und zwei seitliche, in Achsrichtung (30) des Gabelkopfs (14) und weg vom Gewindeschaft (24) sich erstreckende Flügel (48) aufweist, wobei das Druckstück (16) unter Vorspannung in Achsrichtung (30) und damit in Richtung auf den Kugelkopf (26) im Gabelkopf (14) gelagert ist, indem wenigstens einer der Flügel (48) quer zur Achsrichtung (30) des Gabelkopfs (14) geschlitzt ist, so dass ein Schlitz (50) eine Federzunge (52) bildet und das Druckstück (16) dadurch federnd ausgebildet ist und sich über die Federzunge (52) am Gabelkopf (14) abstützt, **dadurch gekennzeichnet, dass** die Federzunge (52) im an den Gabelkopf montierten jedoch nicht zusätzlich axial belasteten Zustand im Bereich ihres an den Flügel (48) angebundenen Endes (56) in Achsrichtung (30) vom Gabelkopf (14) beabstandet ist und dass die Federzunge (52) im zusätzlich axial belasteten Zustand im Bereich ihres an den Flügel (48) angebundenen Endes (56) einen Anschlag (74) bildet und mit diesem Anschlag (74) zur Begrenzung der weitergehenden Verformung oder Auslenkung der Federzunge (52) gegen den Gabelkopf (14) abstützbar ist.

6. Osteosynthesevorrichtung nach einem oder mehreren der vorstehenden Ansprüche, **dadurch gekennzeichnet, dass** das Druckstück (16) und dessen Flügel (48) zur Achsrichtung (30) des Gabelkopfs (14) achssymmetrisch, das heißt rotationssymmetrisch ausgebildet sind.

7. Osteosynthesevorrichtung nach einem oder mehreren der vorstehenden Ansprüche 1-5, **dadurch gekennzeichnet, dass** das Druckstück (16) und dessen Flügel (48) zu einer die Achsrichtung (30) des Gabelkopfs einschließenden Ebene spiegelsymmetrisch ausgebildet sind.

8. Osteosynthesevorrichtung nach einem oder mehreren der vorstehenden Ansprüche, **dadurch gekennzeichnet, dass** der Schlitz (50) an seinem geschlossenen Ende (56) verrundet ausgebildet ist.

9. Osteosynthesevorrichtung nach einem oder mehreren der vorstehenden Ansprüche, **dadurch gekennzeichnet, dass** die Auslenkung der Federzunge (52) beim Einklipsen oder Einrasten oder Eindrehen des Druckstücks in seine Montageposition im Gabelkopf (14) höchstens 1,0 mm, insbesondere höchstens 0,5 mm und weiter insbesondere wenigstens 0,1 mm beträgt.

10. Osteosynthesevorrichtung nach einem oder mehreren der vorstehenden Ansprüche, **dadurch gekennzeichnet, dass** an Außenseiten der Flügel (48) des Druckstücks (16) Vorsprünge (60) oder Ausnehmungen vorhanden sind, die mit Ausnehmungen (62) oder Vorsprüngen an Innenflächen der Schenkel (34) des Gabelkopfs (14) ineinandergreifend zusammenwirken.

11. Osteosynthesevorrichtung nach einem oder mehreren der vorstehenden Ansprüche, **dadurch gekennzeichnet, dass** das Druckstück (16) eine an einer Innenseite eines Schenkels (34) des Gabelkopfs (14) vorhandenen Abstufung untergreift oder in eine dort ausgebildete Ausnehmung eingreift.

12. Osteosynthesevorrichtung nach einem oder mehreren der vorstehenden Ansprüche, **dadurch gekennzeichnet, dass** ein Flügel (48) oder beide Flügel des Druckstücks (16) mehrfach geschlitzt ausgebildet ist/sind, also mehrere Schlitze aufweist/en, die abwechselnd von einer Seite und von einer dieser gegenüberliegenden Seite quer zur Achsrichtung (30) des Gabelkopfs (14) in den Flügel (48) einschneiden.

13. Osteosynthesevorrichtung nach einem oder mehreren der vorstehenden Ansprüche, **dadurch gekennzeichnet, dass** das Druckstück (16) unmittelbar oder mittelbar ein Auflager für einen Verbindungsstab (18) bildet.

14. Osteosynthesevorrichtung nach einem oder mehreren der vorstehenden Ansprüche, **dadurch gekennzeichnet, dass** das Druckstück (16) gegenüber dem Gabelkopf (14) verdrehgesichert ist.

15. Osteosynthesevorrichtung nach einem oder mehreren der vorstehenden Ansprüche, **dadurch gekennzeichnet, dass** die Federzunge (52) am freien Ende (54) in radialer und/oder in axialer Richtung dicker ausgebildet ist als am geschlossenen Ende (56).

## Claims

1. An osteosynthesis device having a bone screw (12) which has a threaded shaft (24) and a ball head (26), and having a fork head (14) which has a groove (32) and two legs (34), the ball head (26) being pivotably supported in the fork head (14), and having a pressure piece (16) which is seated on the ball head (26), the pressure piece (16) being braced on the fork head (14) and having two lateral fins (48) extending in the axial direction (30) of the fork head (14) and away from the threaded shaft (24), wherein the pressure piece (16) is supported in the fork head (14) with prestressing in the axial direction (30) and thus in the direction toward the ball head (26) by at least one of the fins (48) being slit transversely to the axial direction (30) of the fork head (14), so that a slit (50) forms a spring tongue (52), and the pressure piece (16) is thereby resiliently embodied and is braced via the spring tongue (52) on the fork head (14) ;
**characterized in that** a protrusion (70) protrudes in the axial direction (30) into the slit (50), and that the protrusion (70), for limiting deformation or deflection of the spring tongue (52), forms a stop (72), acting in the axial direction (30), for the spring tongue (52).

2. The osteosynthesis device of claim 1, **characterized in that** the protrusion (70) is embodied opposite, in the axial direction (30), the spring tongue (52) on the pressure piece (16).

3. The osteosynthesis device of claim 1, **characterized in that** the protrusion (70) is embodied on the spring tongue (52) .

4. The osteosynthesis device of claim 1, 2 or 3, **characterized in that** the protrusion (70) is embodied on a free end (54) of the spring tongue (52) or on a region on the pressure piece opposite the free end of the spring tongue in the axial direction.

5. An osteosynthesis device having a bone screw (12) which has a threaded shaft (24) and a ball head (26), and having a fork head (14) which has a groove (32) and two legs (34), the ball head (26) being pivotably supported in the fork head (14), and having a pressure piece (16) which is seated on the ball head (26), the pressure piece (16) being braced on the fork head (14) and having two lateral fins (48) extending in the axial direction (30) of the fork head (14) and away from the threaded shaft (24), wherein the pressure piece (16) is supported in the fork head (14) with prestressing in the axial direction (30) and thus in the direction toward the ball head (26) by at least one of the fins (48) being slit transversely to the axial direction (30) of the fork head (14), so that a slit (50) forms a spring tongue (52), and the pressure piece (16) is thereby resiliently embodied and is braced via the spring tongue (52) on the fork head (14); **characterized in that** the spring tongue (52), in the state in which it is installed on the fork head but is not additionally axially stressed, is spaced apart in the vicinity of its end (56), joined to the fin (48), from the fork head (14) in the axial direction (30); and that the spring tongue (52), in the additionally axially stressed state, in the vicinity of its end (56) joined to the fin (48) forms a stop (74) and can be braced with the stop (74) against the fork head (14) for limiting more-extensive deformation or deflection of the spring tongue (52) .

6. The osteosynthesis device of one or more of the foregoing claims, **characterized in that** the pressure piece (16) and its fin (48) are embodied axially symmetrically, or in other words rotationally symmetrically, to the axial direction (30) of the fork head (14).

7. The osteosynthesis device of one or more of the foregoing claims 1-5, **characterized in that** the pressure piece (16) and its fin (48) are embodied mirror-symmetrically to a plane that includes the axial direction (30) of the fork head.

8. The osteosynthesis device of one or more of the foregoing claims, **characterized in that** the slit (50) is embodied in rounded fashion on its closed end (56).

9. The osteosynthesis device of one or more of the foregoing claims, **characterized in that** the deflection of the spring tongue (52), upon the clasping or latching or screwing in of the pressure piece into its installed position in the fork head (14), amounts to at most 1.0 mm, in particular at most 0.5 mm, and more particularly at least 0.1 mm.

10. The osteosynthesis device of one or more of the foregoing claims, **characterized in that** on outer sides of the fins (48) of the pressure piece (16), there are protrusions (60) or recesses, which cooperate in intermeshing fashion with recesses (62) or protrusions on inside surfaces of the legs (34) of the fork head (14).

11. The osteosynthesis device of one or more of the foregoing claims, **characterized in that** the pressure piece (16) engages a graduation, located on an inner side of a leg (34) of the fork head (14), from beneath or engages the inside of a recess embodied there.

12. The osteosynthesis device of one or more of the foregoing claims, **characterized in that** one fin (48) or both fins of the pressure piece (16) are embodied with multiple slits, or in other words have a plurality of slits, which are cut inward into the fin or fins (48) in alternation from one side and a side opposite that side transversely to the axial direction (30) of the fork head (14).

13. The osteosynthesis device of one or more of the foregoing claims, **characterized in that** the pressure piece (16) directly or indirectly forms a support for a connecting rod (18).

14. The osteosynthesis device of one or more of the foregoing claims, **characterized in that** the pressure piece (16) is secured against relative rotation with respect to the fork head (14).

15. The osteosynthesis device of one or more of the foregoing claims, **characterized in that** the spring tongue (52) is embodied as thicker on the free end (54) in the radial and/or axial direction than at the closed end (56).

## Revendications

1. Dispositif d'ostéosynthèse comprenant une vis à os (12) présentant une tige filetée (24) et une tête sphérique (26), et une tête fourchue (14) présentant une rainure (32) et ayant deux branches (34), ladite tête sphérique (26) étant logée à pivotement à l'intérieur de la tête fourchue (14), ainsi qu'une pièce de pression (16) qui repose sur la tête sphérique (26), dans lequel ladite pièce de pression (16) prend appui sur la tête fourchue (14) et présente deux ailes (48) latérales s'étendant dans la direction axiale (30) de la tête fourchue (14) et dans la direction opposée à la tige filetée (24), dans lequel ladite pièce de pression (16) est logée dans la tête fourchue (14) en étant précontrainte dans la direction axiale (30) et ainsi en direction de la tête sphérique (16) par le fait que l'une au moins des ailes (48) est fendue transversalement à la direction axiale (30) de la tête fourchue (14), de sorte qu'une fente (50) forme une languette élastique (52) et que la pièce de pression (16) est configurée ainsi de manière élastique et prend appui sur la tête fourchue (14) par ladite languette élastique (52), **caractérisé par le fait qu'**une projection (70) fait saillie dans la direction axiale (30) dans ladite fente (50) et que la projection (70) forme une butée (72) pour la languette élastique (52) pour limiter une déformation ou déviation de la languette élastique (52).

2. Dispositif d'ostéosynthèse selon la revendication 1, **caractérisé par le fait que** la projection (70) est réalisée sur la pièce de pression (16) en regard de la languette élastique (52) dans la direction axiale (30).

3. Dispositif d'ostéosynthèse selon la revendication 1, **caractérisé par le fait que** la projection (70) est réalisée sur la languette élastique (52).

4. Dispositif d'ostéosynthèse selon la revendication 1, 2 ou 3, **caractérisé par le fait que** la projection (70) est réalisée sur la pièce de pression à une extrémité libre (54) de la languette élastique (52) ou sur une zone située en regard de l'extrémité libre de la languette élastique dans la direction axiale.

5. Dispositif d'ostéosynthèse comprenant une vis à os (12) présentant une tige filetée (24) et une tête sphérique (26), et une tête fourchue (14) présentant une rainure (32) et ayant deux branches (34), ladite tête sphérique (26) étant logée à pivotement à l'intérieur de la tête fourchue (14), ainsi qu'une pièce de pression (16) qui repose sur la tête sphérique (26), dans lequel ladite pièce de pression (16) prend appui sur la tête fourchue (14) et présente deux ailes (48) latérales s'étendant dans la direction axiale (30) de la tête fourchue (14) et dans la direction opposée à la tige filetée (24), dans lequel ladite pièce de pression (16) est logée dans la tête fourchue (14) en étant précontrainte dans la direction axiale (30) et ainsi en direction de la tête sphérique (16) par le fait que l'une au moins des ailes (48) est fendue transversalement à la direction axiale (30) de la tête fourchue (14), de sorte qu'une fente (50) forme une languette élastique (52) et que la pièce de pression (16) est configurée ainsi de manière élastique et prend appui sur la tête fourchue (14) par ladite languette élastique (52), **caractérisé par le fait que** la languette élastique (52), à l'état où elle est montée sur la tête fourchue mais n'est pas chargée en sus axialement, est espacée de la tête fourchue (14) dans la direction axiale (30) au niveau de son extrémité (56) attachée à l'aile (48), et que la languette élastique (52), à l'état où elle est chargée en sus axialement, forme une butée (74) au niveau de son extrémité (56) attachée à l'aile (48) et peut être appuyée par cette butée (74) contre la tête fourchue (14) pour limiter la déformation ou déviation continuée de la languette élastique (52).

6. Dispositif d'ostéosynthèse selon l'une ou plusieurs des revendications précédentes, **caractérisé par le fait que** la pièce de pression (16) et ses ailes (48) sont réalisées à symétrie axiale par rapport à la direction axiale (30) de la tête fourchue (14), c'est-à-dire à symétrie de révolution.

7. Dispositif d'ostéosynthèse selon l'une ou plusieurs des revendications précédentes 1 à 5, **caractérisé par le fait que** la pièce de pression (16) et ses ailes (48) sont réalisées à symétrie spéculaire par rapport à un plan comprenant la direction axiale (30) de la tête fourchue.

8. Dispositif d'ostéosynthèse selon l'une ou plusieurs des revendications précédentes, **caractérisé par le fait que** ladite fente (50) est réalisée de manière arrondie à son extrémité fermée (56).

9. Dispositif d'ostéosynthèse selon l'une ou plusieurs des revendications précédentes, **caractérisé par le fait que**, lorsque la pièce de pression est clipsée ou enclenchée ou insérée en tournant dans sa position de montage dans la tête fourchue (14), la déviation de la languette élastique (52) est de 1,0 mm tout au plus, en particulier de 0,5 mm tout au plus et plus particulièrement d'au moins 0,1 mm.

10. Dispositif d'ostéosynthèse selon l'une ou plusieurs des revendications précédentes, **caractérisé par le fait que** des projections (60) ou évidements sont présent(e)s sur des faces extérieures des ailes (48) de la pièce de pression (16), qui agissent de concert à engagement avec des évidements (62) ou projections présent(e)s sur des faces intérieures des branches (34) de la tête fourchue (14) .

11. Dispositif d'ostéosynthèse selon l'une ou plusieurs des revendications précédentes, **caractérisé par le fait que** la pièce de pression (16) se prend sous un gradin existant sur une face intérieure d'une branche (34) de la tête fourchue (14) ou s'engage dans un évidement y ménagé.

12. Dispositif d'ostéosynthèse selon l'une ou plusieurs des revendications précédentes, **caractérisé par le fait qu'**une aile (48) ou les deux ailes de la pièce de pression (16) est/sont réalisée(s) de manière à être fendue(s) plusieurs fois, présente(nt) donc une pluralité de fentes qui coupent dans l'aile (48) transversalement à la direction axiale (30) de la tête fourchue (14), alternativement depuis un côté et depuis un côté opposé à celui-ci.

13. Dispositif d'ostéosynthèse selon l'une ou plusieurs des revendications précédentes, **caractérisé par le fait que** la pièce de pression (16) forme directement ou indirectement un appui pour une tige de liaison (18).

14. Dispositif d'ostéosynthèse selon l'une ou plusieurs des revendications précédentes, **caractérisé par le fait que** la pièce de pression (16) est bloquée en rotation par rapport à la tête fourchue (14).

15. Dispositif d'ostéosynthèse selon l'une ou plusieurs des revendications précédentes, **caractérisé par le fait que** la languette élastique (52) est réalisée de manière à être plus épaisse dans la direction radiale et/ou axiale à l'extrémité libre (54) qu'à l'extrémité fermée (56).
